# EUROPÄISCHE PATENTSCHRIFT

(11) **EP 3 944 814 B1**
(45) Veröffentlichungstag und Bekanntmachung des Hinweises auf die Patenterteilung: **07.12.2022**
(21) Anmeldenummer: 20188711.4
(22) Anmeldetag: 30.07.2020
(51) Int. Cl.: A61B 5/15, B01L 3/00, A61B 10/00

(54) **VORRICHTUNG ZUR ENTNAHME EINER FLÜSSIGKEITSPROBE**
DEVICE FOR TAKING A LIQUID SAMPLE
DISPOSITIF DE PRÉLÈVEMENT D'UN ÉCHANTILLON LIQUIDE

(43) Veröffentlichungstag der Anmeldung: 02.02.2022
(73) Patentinhaber: Protzek Biotec GmbH, 4415 Lausen (CH)
(72) Erfinder: Protzek, Christoph, 79539 Lörrach (DE); Weinmann, Alexander, 79100 Freiburg (DE)
(74) Vertreter: Patentanwälte Dörner & Kötter PartG mbB

(56) Entgegenhaltungen:
- EP-A1- 1 752 755
- WO-A1-2014/172247
- WO-A2-02/097389
- DE-U1-202016 104 645
- US-A1- 2018 314 792

## Beschreibung

Die Erfindung betrifft eine Vorrichtung zur Entnahme einer Flüssigkeitsprobe des menschlichen oder tierischen Körpers, insbesondere einer Blutprobe nach dem Patentanspruch 1.

Dokument US 2018/314792 A1 offenbart eine Vorrichtung zur Probenentnahme einer Blutprobe des menschlichen oder tierischen Körpers, umfassend eine Trägerplatte, die einen ersten Abschnitt aufweist, an dem ein zweiter Abschnitt über ein Scharnier schwenkbar verbunden ist, wobei an dem ersten Abschnitt wenigstens zwei Zellulosezähne befestigt sind.

Die Dried-Blood-Spot (DBS-) Analyse hat sich in der jüngsten Zeit als Probenahmeverfahren in bioanalytischen, klinischen und pharmazeutischen Anwendungen bewährt. Gegenüber der herkömmlichen Entnahmeverfahren von Vollblut-, Plasma- oder Serumproben erweist sich dieses Verfahren in Bezug auf die Probenentnahme sowie auch deren Versand und Lagerung als deutlich weniger invasiv und kostengünstig. Mittels eines kleinen Fingerstichs kann die Probe durch minimal geschultes Personal oder auch durch den Patienten selbst entnommen und auf einen DBS-Träger aufgegeben werden. Bis zur eigentlichen Analyse ist die Probe in dem DBS-Probenträger während des Versands und der Lagerung stabil und vor äußeren Einwirkungen geschützt.

Nachteilig an der vorbekannten DBS-Analyse ist, dass das eingesetzte Filterpapier lediglich eine passive Unterstützung für die Blutentnahme bietet. Eine genaue Volumenmessung für eine solche Analyse erfordert nachgeschaltete Bearbeitungsschritte, die für den Benutzer zeit- und kostenaufwändig sind.

Hier will die Erfindung Abhilfe schaffen. Der Erfindung liegt die Aufgabe zugrunde, eine Vorrichtung zur Entnahme einer Flüssigkeitsprobe bereitzustellen, die eine aufwandminimierte Probenentnahme bei gleichzeitiger Vorgabe eines zu entnehmenden Volumens ermöglicht. Gemäß der Erfindung wird diese Aufgabe durch eine Vorrichtung mit den Merkmalen des Patentanspruchs 1 gelöst.

Mit der Erfindung ist eine Vorrichtung zur Entnahme einer Flüssigkeitsprobe bereitgestellt, die eine aufwandminimierte Probenentnahme bei gleichzeitiger Vorgabe eines zu entnehmenden Volumens ermöglicht. Dadurch, dass die Vorrichtung eine Trägerplatte umfasst, die einen ersten Abschnitt aufweist, an dem ein zweiter Abschnitt über ein Scharnier schwenkbar verbunden ist, wobei an dem ersten Abschnitt wenigstens zwei Zellulosezähne befestigt sind, die mit definierten Flüssigkeits-Aufnahmevolumen in den Bereich des zweiten Abschnitts hineinragen, ist eine einfache Probenentnahme mittels der Zellulosezähne mit ihren definierten Flüssigkeits-Aufnahmevolumen ermöglicht. Durch Verschwenken des zweiten Abschnitts sind die Zellulosezähne frei zugänglich. Nach Eintauchen der Zellulosezähne in eine Flüssigkeitsprobe, beispielsweise eine Blutprobe, wird das definierte Aufnahmevolumen an Flüssigkeit durch die Zellulosezähne aufgenommen. Es kann auch direkt Kapillarblut von einer Fingerkuppe von eine Zellulosezahn aufgenommen werden, der sich von seinem verjüngten freien Ende her vollsaugt.

Durch die Entnahme eines einzelnen Zellulosezahns liegt die Flüssigkeitsprobe sodann in einem über die Abmessungen des Zellulosezahns vordefinierten Volumen zur weiteren Analyse vor. Je nach Größe des Zellulosezahns ist auch eine Extraktion der aufgesaugten definierten Flüssigkeitsprobe, insbesondere einer Blutprobe, mit Hilfe eines Autosamplers möglich.

In Weiterbildung der Erfindung ist der zweite Abschnitt in Form eines Rahmens ausgebildet, der gemeinsam mit dem ersten Abschnitt ein Fenster begrenzt. Hierdurch bleiben die Zellulosezähne auch bei nicht verschwenktem zweiten Abschnitt sichtbar. Bevorzugt ist das Fenster mit einer transparenten Folie verdeckt.

In Weiterbildung der Erfindung ist die Trägerplatte aus Pappe oder Karton hergestellt, wobei das wenigstens eine Scharnier durch eine Knicklinie gebildet ist. Hierdurch ist eine kostengünstige Herstellung der Vorrichtung erzielt, welche nach Gebrauch umweltfreundlich zu entsorgen ist.

In Ausgestaltung der Erfindung ist der Rahmenabschnitt in Richtung seiner Ausgangslage in Flucht mit der Trägerplatte vorgespannt. Hierzu ist das wenigstens eine Scharnier vorzugsweise mit einer dieses überspannenden Folie flächig verklebt. Dabei ist die Folie derart ausgebildet, dass sie nach einem Umklappen reversibel in ihre Ausgangslage zurückkehrt. Vorteilhaft ist die Folie transparent ausgebildet und überdeckt zugleich das durch den zweiten Abschnitt begrenzte Fenster.

In weiterer Ausgestaltung der Erfindung sind die wenigstens zwei Zellulosezähne über jeweils eine Perforationslinie begrenzt, über die sie definiert und unabhängig voneinander abtrennbar sind. Hierdurch ist eine einfache Abtrennung eines einzelnen Zellulosezahns mit dem von diesen aufgenommenen definierten Flüssigkeitsvolumen ermöglicht. Vorteilhaft weisen die wenigstens zwei Zellulosezähne identische Flächen mit identischen Volumina auf. Vorzugsweise betragen die Flächen zwischen 35 mm² und 64 mm² und weisen ein Volumen von zwischen 18 µl und 33 µl auf. Das von einem Zellulosezahn aufgenommene Probenvolumen bestimmt sich durch die Abmessungen des Zahns (Zahnfläche und -dicke), der Zellulosequalität sowie der Art der Probe (Matrix).

In Weiterbildung der Erfindung sind die wenigstens zwei Zellulosezähne Bestandteil eines Zellulosevlieses, das mit der Trägerplatte verbunden ist. Hierdurch ist die Saugwirkung der Zellulosezähne unterstützt, wobei überschüssiges Flüssigkeitsvolumen von dem Zellulosevlies aufgenommen werden kann. Vorzugsweise ist das Zellulosevlies in Form eines Filterpapiers ausgebildet, dessen Kontur einfach durch einen Stanzvorgang herstellbar ist.

In weiterer Ausgestaltung der Erfindung sind die wenigstens zwei Zellulosezähne an ihrem freien Ende dreieck- oder trapezförmig sich verjüngend ausgebildet. Hierdurch ist ein minimaler Kontakt eines Zellulosezahns mit der Probeflüssigkeit zu deren Aufnahme erzielt.

In Weiterbildung der Erfindung ist die Trägerplatte schwenkbar mit einer Basisplatte verbunden, die derart dimensioniert ist, dass sie bei Anlage an der Trägerplatte diese vollständig, vorzugweise bündig überdeckt. Hierdurch ist ein guter Schutz der Zellulosezähne vor, sowie auch nach einer Probenentnahme gewährleistet.

In Ausgestaltung der Erfindung sind Trägerplatte und Basisplatte Bestandteil eines einteiligen Pappen- oder Kartonagenzuschnitts und sind über eine Knicklinie schwenkbar verbunden. Hierdurch ist eine einfache und umweltfreundliche Herstellung ermöglicht.

In weiterer Ausgestaltung der Erfindung ist in der Basisplatte eine wannenförmige Einbuchtung zur Aufnahme eines Trockenmittels angeordnet. Hierdurch ist eine zügige, gleichmäßige Trocknung der mit Flüssigkeitsproben versehenen Zellulosezähne ermöglicht.

In weiterer Ausgestaltung der Erfindung ist die Einbuchtung außerhalb des Bereichs angeordnet, der im zusammengeklappten Zustand von Basis- und Trägerplatte von dem zweiten Abschnitt und/oder den wenigstens zwei Zellulosezähnen überdeckt ist. Hierdurch ist einer Kontaminierung von Zellulosezähnen durch Trockenmittel entgegengewirkt.

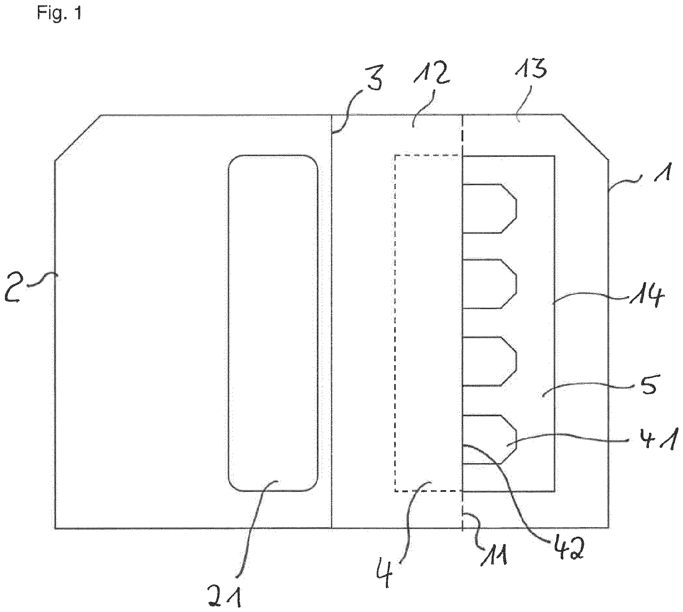

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Die einzige Figur 1 zeigt die schematische Darstellung einer Vorrichtung zur Probenentnahme einer Flüssigkeit.

Die als Ausführungsbeispiel gewählte Vorrichtung ist als klappbare Testkarte ausgebildet. Diese umfasst eine Trägerplatte 1, die mit einer Basisplatte 2 schwenkbar verbunden ist. Trägerplatte 1 und Basisplatte 2 sind aus einem gemeinsamen, im Wesentlichen rechteckigen Kartonagenzuschnitt mit zwei gegenüberliegenden abgeschrägten Ecken gebildet und über eine mittig angeordnete, erste Knickfalz 3, die ein Scharnier ausbildet, schwenkbar verbunden. Trägerplatte 1 und Basisplatte 2 weisen somit jeweils eine abgeschrägte Ecke auf.

Die Trägerplatte 1 ist wiederum durch eine zweite, etwa mittig angeordnete Knickfalz 11 in einen ersten Abschnitt 12 und einen zweiten Abschnitt 13 unterteilt, die über diese zweite Knickfalz 11 schwenkbar verbunden sind. In den zweiten Abschnitt 13 ist ein im Wesentlichen rechteckiges Fenster 14 eingebracht, dessen eine Längsseite entlang der zweiten Knickfalz 11 verläuft.

An dem ersten Abschnitt ist ein im Wesentlichen rechteckiges Vlies 4 befestigt, das an einer Längsseite beabstandet zueinander vier Zellulosezähne 41 aufweist, mit denen das Vlies 4 in den Bereich des Fensters 14 hineinragt. Die Zellulosezähne 41 sind jeweils an ihrem freien Ende trapezförmig bzw. satteldachförmig ausgebildet und weisen im Ausführungsbeispiel eine Fläche von 48 mm² mit einem Flüssigkeitsaufnahmevolumen von 20 µl auf. Entlang der zweiten Knickfalz 11 sind die Zellulosezähne 41 mit einer Perforationslinie 42 versehen, über die sie abtrennbar sind.

In die Basisplatte 2 ist auf ihrer der Trägerplatte 1 zugewandten hälftigen Seite eine wannenförmige Einbuchtung 21 zur Aufnahme von - nicht dargestelltem - Trockenmaterial eingebracht. Das Trockenmaterial kann beispielsweise durch ein mit Silikat gefülltes Säckchen gebildet sein, das in die Einbuchtung 21 eingeklebt ist.

Trägerplatte 1 und Basisplatte 2 sind auf ihrer der Einsenkung abgewandten Seite über eine flächig verklebte transparente, widerstandsfähige Folie 5 überdeckt, die derart ausgebildet ist, dass sie nach Verschwenken des zweiten Abschnitts 13 der Trägerplatte 1 in ihre Ursprungsposition zurückkehrt. Über die flächig aufgeklebte Folie 5 ist zugleich das Vlies 4 auf dem ersten Abschnitt 12 befestigt. Im Bereich des Fensters 14 ist die Folie 5 glatt und insbesondere nicht klebend ausgebildet. Außerhalb des Fensters 5 kann die Folie auch als beschriftetes und/oder zu beschriftendes Etikett ausgebildet sein.

Im zugeklappten Zustand liegt die Trägerplatte 1 auf der Basisplatte 2 auf, wobei der erste Abschnitt 12 der Trägerplatte 1 die wannenförmige Einbuchtung 21 verdeckt. Die Zellulosezähne 41 befinden sich dabei außerhalb des Bereichs der Einbuchtung 21 und sind durch die Basisplatte 2 einerseits und die das Fenster 14 überspannende transparente Folie 5 andererseits geschützt.

Zur Aufnahme von Proben werden Trägerplatte 1 und Basisplatte 2 auseinandergeschwenkt, wonach der das Fenster 14 aufweisende zweite Abschnitt 13 nach hinten geklappt und gehalten wird. Die Zellulosezähne 41 ragen nun frei hervor und können in die zu beprobende Flüssigkeit getaucht werden. Wird der zweite Abschnitt 13 losgelassen, wird er durch die vorgespannte transparente Folie in seine Ursprungslage zurückbewegt. Die mit der Probenflüssigkeit versehenen Zellulosezähne 41 befinden sich nun geschützt innerhalb des mit der Folie 5 überdeckten Fensters 14 und sind im zusammengeklappten Zustand der Testkarte zusätzlich durch die Basisplatte 2 geschützt.

In einer weiteren Ausführungsform können die Zellulosezähne auch an einem verschiebbar angeordneten Tragelement befestigt sein, über das sie temporär in dem Bereich des Fensters verschiebbar sind. Dabei kann das Trägerelement beispielsweise durch einen Zellulosestreifen gebildet sein, an dem die Zellulosestreifen angeformt sind. Ein solcher mit Zähnen versehener Zellulosestreifen ist aufwandminimiert durch Ausstanzen aus einem Zelluloseblatt herstellbar. Nach Aufnahme von Probenflüssigkeit durch die Zähne kann das Trägerelement mit den Zähnen in einen geschützten Bereich verschoben werden.

Durch die erfindungsgemäße Vorrichtung ist ein einfach zu handhabendes Set zur Flüssigkeitenentnahme, insbesondere zur Blutentnahme bereitgestellt, das definiert Sample-Proben für nachgerückte Analysen aufnehmen kann, wobei ein maximaler Kontaminations- und Trocknungsschutz durch Zuklappen der Testkarte gegeben ist. Durch die flächendefinierten Zellulosezähne 41 des Vlieses 4 stehen mehrere (im Ausführungsbeispiel vier) Proben gleichen Volumens zur Analyse zur Verfügung, die mittels einer Pinzette definiert entlang der Perforationslinie 42 extrahiert und weiterverarbeitet werden können. Vorzugsweise ist die Form der Testkarte derart dimensioniert und ausgeführt, dass sie von einem Autosampler aufgenommen und automatisiert abgearbeitet werden kann.

## Patentansprüche

1. Vorrichtung zur Probenentnahme einer Flüssigkeit des menschlichen oder tierischen Körpers, insbesondere einer Blutprobe, umfassend eine Trägerplatte (1), die einen ersten Abschnitt (12) aufweist, an dem ein zweiter Abschnitt (13) über ein Scharnier schwenkbar verbunden ist, wobei an dem ersten Abschnitt (12) wenigstens zwei Zellulosezähne (41) befestigt sind, die ein definiertes Flüssigkeits-Aufnahmevolumen aufweisen und in den Bereich des zweiten Abschnitts (13) hineinragen.

2. Vorrichtung nach Anspruch 1, **dadurch gekennzeichnet, dass** der zweite Abschnitt (13) in Form eines Rahmens ausgebildet ist, der gemeinsam mit dem ersten Abschnitt ein Fenster (14) begrenzt.

3. Vorrichtung nach Anspruch 1 oder 2, **dadurch gekennzeichnet, dass** die Trägerplatte (1) aus Pappe oder Karton hergestellt ist, wobei das wenigstens eine Scharnier durch eine Knickfalz (11) gebildet ist.

4. Vorrichtung nach einem der vorgenannten Ansprüche, **dadurch gekennzeichnet, dass** der zweite Abschnitt (13) in Richtung seiner Ausgangslage in Flucht mit der Trägerplatte (1), vorzugsweise über eine das wenigstens eine Scharnier überspannende, flächig verklebte Folie (5), vorgespannt ist.

5. Vorrichtung nach einem der vorgenannten Ansprüche, **dadurch gekennzeichnet, dass** die wenigstens zwei Zellulosezähne (41) über jeweils eine Perforationslinie (42) begrenzt sind, über die sie definiert und unabhängig voneinander abtrennbar sind.

6. Vorrichtung nach einem der vorgenannten Ansprüche, **dadurch gekennzeichnet, dass** die wenigstens zwei Zellulosezähne (41) identische Flächen, vorzugsweise zwischen 35 mm² und 64 mm², mit identischen Volumina, vorzugsweise zwischen 18µl und 33 µl aufweisen.

7. Vorrichtung nach einem der vorgenannten Ansprüche, **dadurch gekennzeichnet, dass** die wenigstens zwei Zellulosezähne (41) Bestandteil eines Zellulosevlieses (4) sind, das mit der Trägerplatte (1) verbunden ist.

8. Vorrichtung nach einem der vorgenannten Ansprüche, **dadurch gekennzeichnet, dass** die wenigstens zwei Zellulosezähne (41) an ihrem freien Ende dreieck- oder trapezförmig sich verjüngend ausgebildet sind.

9. Vorrichtung nach einem der vorgenannten Ansprüche, **dadurch gekennzeichnet, dass** die Trägerplatte (1) schwenkbar mit einer Basisplatte (2) verbunden ist, die derart dimensioniert ist, dass sie bei Anlage an der Trägerplatte (1) diese vollständig, vorzugsweise bündig überdeckt.

10. Vorrichtung nach Anspruch 9 **dadurch gekennzeichnet, dass** Trägerplatte (1) und Basisplatte (2) Bestandteil eines einteiligen Pappen- oder Kartonagenzuschnitts sind und über eine Knicklinie (3) schwenkbar verbunden sind.

11. Vorrichtung nach Anspruch 9 oder 10, **dadurch gekennzeichnet, dass** in der Basisplatte (2) eine wannenförmige Einbuchtung (21) zur Aufnahme eines Trockenmittels angeordnet ist.

12. Vorrichtung nach Anspruch 11, **dadurch gekennzeichnet, dass** die Einbuchtung (21) außerhalb des Bereichs angeordnet ist, der in zusammengeklappten Zustand von Basis (2)- und Trägerplatte (1) von dem zweiten Abschnitt (13) und/oder den wenigstens zwei Zellulosezähnen (41) überdeckt ist.

## Claims

1. Device for taking a sample of a liquid of the human or animal body, in particular a blood sample, comprising a carrier plate (1), which has a first section (12), to which a second section (13) is pivotably connected via a hinge, wherein at least two cellulose teeth (41) are attached to the first section (12), which have a defined liquid receiving volume and project into the region of the second section (13).

2. Device according to claim 1, **characterised in that** the second section (13) is designed in the form of a frame, which, together with the first section, delimits a window (14).

3. Device according to claim 1 or 2, **characterised in that** the carrier plate (1) is made of cardboard or paperboard, wherein the at least one hinge is formed by a fold (11).

4. Device according to one of the previous claims, **characterised in that** the second section (13) is pretensioned in the direction of its initial position in alignment with the carrier plate (1), preferably via a film (5) which is bonded over the surface and spans the at least one hinge.

5. Device according to one of the previous claims, **characterised in that** the at least two cellulose teeth (41) are each delimited via a perforation line (42), via which they are defined and can be separated independently from one another.

6. Device according to one of the previous claims, **characterised in that** the at least two cellulose teeth (41) have identical areas, preferably between 35 mm² and 64 mm², with identical volumes, preferably between 18 µl and 33 µl.

7. Device according to one of the previous claims, **characterised in that** the at least two cellulose teeth (41) are parts of a cellulose fleece (4), which is connected to the carrier plate (1).

8. Device according to one of the previous claims, **characterised in that** the at least two cellulose teeth (41) are of triangular or trapezoidal tapering design at their free end.

9. Device according to one of the previous claims, **characterised in that** the carrier plate (1) is pivotably connected to a base plate (2), which is dimensioned in such a manner that when it rests against the carrier plate (1), it covers the latter completely, preferably flush.

10. Device according to claim 9, **characterised in that** carrier plate (1) and base plate (2) are part of a one-piece cardboard or cardboard blank and are pivotably connected via a fold line (3).

11. Device according to claim 9 or 10, **characterised in that** a trough-shaped indentation (21) for receiving a desiccant is arranged in the base plate (2).

12. Device according to claim 11, **characterised in that** the indentation (21) is arranged outside of the area, which, in the folded state of base plate (2) and carrier plate (1), is covered by the second section (13) and/or the at least two cellulose teeth (41).

## Revendications

1. Dispositif de prélèvement d'un échantillon de liquide en provenance du corps humain ou d'un corps d'animal, notamment d'un échantillon sanguin, comprenant une plaque support (1) qui présente un premier segment (12) auquel est raccordé de façon pivotante, via une charnière, un second segment (13), sachant que contre le premier segment (12) sont fixées au moins deux dents en cellulose (41) pouvant absorber un volume défini de liquide et qui font saillie dans la zone du second segment (13).

2. Dispositif selon la revendication 1, **caractérisé en ce que** le deuxième segment (13) est configuré en forme de cadre délimitant, avec le concours du premier segment, une fenêtre (14).

3. Dispositif selon la revendication 1 ou 2, **caractérisé en ce que** la plaque support (1) est fabriquée en carton-pâte ou carton, sachant qu'au moins une charnière est formée par une pliure (11).

4. Dispositif selon l'une des revendications précédentes, **caractérisé en ce que** le deuxième segment (13) est précontraint - en direction de sa position de départ en alignement avec la plaque support (1) - de préférence via un film (5) collé à plat et recouvrant tendu au moins une charnière.

5. Dispositif selon l'une des revendications précédentes, **caractérisé en ce qu'**au moins deux dents (41) en cellulose sont limitées via une ligne de perforation (42) respective via laquelle elles sont définies et détachables l'une indépendamment de l'autre.

6. Dispositif selon l'une des revendications précédentes, **caractérisé en ce qu'**au moins deux dents (41) en cellulose présentent des surfaces identiques, comprises de préférence entre 35 mm² et 64 mm², et des volumes identiques compris de préférence entre 18 µl et 33 µl.

7. Dispositif selon l'une des revendications précédentes, **caractérisé en ce qu'**au moins deux dents (41) en cellulose font partie d'un non-tissé (4) en cellulose qui est relié à la plaque support (1).

8. Dispositif selon l'une des revendications précédentes, **caractérisé en ce qu'**au moins deux dents (41) en cellulose sont configurées se rétrécissant, à leur extrémité libre, en triangle ou en trapèze.

9. Dispositif selon l'une des revendications précédentes, **caractérisé en ce que** la plaque support (1) est reliée pivotante avec une plaque de base (2), plaque de base qui est dimensionnée de sorte à recouvrir la plaque support (1) entièrement, de préférence à ras, lorsqu'elle applique dessus.

10. Dispositif selon la revendication 9, **caractérisé en ce que** la plaque support (1) et la plaque de base (2) font partie intégrante d'une découpe monobloc dans du carton-pâte ou du cartonnage et qu'elles sont reliées entre elles de manière pivotante via une ligne de pliure (3).

11. Dispositif selon la revendication 9 ou 10, **caractérisé en ce que** dans la plaque de base (2) est disposée une déformation concave (21) en forme de bac destinée à recevoir un siccatif.

12. Dispositif selon la revendication 11, **caractérisé en ce que** la déformation concave (21) est disposée en dehors de la zone qui - lorsque la plaque de base (2) et la plaque support (1) sont à l'état replié - est recouverte par le deuxième segment (13) et/ou par au moins les deux dents (41) en cellulose.
